# EUROPÄISCHE PATENTSCHRIFT

(11) **EP 3 209 265 B1**
(45) Veröffentlichungstag und Bekanntmachung des Hinweises auf die Patenterteilung: **06.01.2021**
(21) Anmeldenummer: 15791510.9
(22) Anmeldetag: 13.10.2015
(51) Int. Cl.: A61K 6/00, A61C 5/82, H04R 25/00

(54) **FRÄSROHLING ZUR HERSTELLUNG VON MEDIZINTECHNISCHEN FORMTEILEN**
MILLING BLANK FOR PRODUCTION OF MEDICAL MOULDED PARTS
FLAN DE FRAISAGE DESTINÉ À LA FABRICATION DE PIÈCES MOULÉES À USAGE MÉDICAL

(30) Priorität: 14.10.2014 DE 102014114895
(43) Veröffentlichungstag der Anmeldung: 30.08.2017
(73) Patentinhaber: PRO3DURE MEDICAL GMBH, 44227 Dortmund (DE)
(72) Erfinder: KLARE, Martin, 44227 Dortmund (DE); GISCHER, Frank, 58708 Menden (DE)
(74) Vertreter: Schulz Junghans Patentanwälte PartGmbB
(86) Internationale Anmeldenummer: PCT/EP2015/073636
(87) Internationale Veröffentlichungsnummer: WO 2016/059029

(56) Entgegenhaltungen:
- EP-A1- 0 410 034
- EP-A2- 1 702 633
- DE-A1- 10 011 665
- DE-A1- 10 147 125
- DE-A1- 19 961 341
- DE-A1-102012 022 693

## Beschreibung

Die Erfindung betrifft einen Fräsrohling zur Herstellung von medizintechnischen Formteilen, insbesondere von Dentalschienen oder Ohrpassstücken, sowie ein Verfahren zur Herstellung eines solchen Rohlings.

Rohlinge zur Herstellung von medizintechnischen Formteilen der eingangs genannten Art sind in vielfältiger Ausgestaltung aus dem Stand der Technik bekannt. Sowohl dentale Schienen als auch Ohrpassstücke werden gegenwärtig im Wesentlichen mittels zwei unterschiedlicher Verfahren des Standes der Technik hergestellt.

Bei dem ersten aus dem Stand der Technik bekannten Verfahren wird dabei in einem ersten Schritt ein Abdruck des Positivs (Zahnkranz oder Gehörgang) genommen. Anschließend wird im Falle einer dentalen Schiene aus dem Abdruck ein Positiv-Gipsmodell hergestellt, auf welchem dann z.B. eine Schiene (Negativ) tiefgezogen oder mittels z.B. Streutechnik ein 2-Komponentenmaterial aufgetragen und anschließend ausgehärtet wird. Beim so bezeichneten PNP-Verfahren (Positiv-Negativ-Positiv) zur Herstellung von Ohrpassstücken nimmt der Hörgeräteakustiker in einem ersten Schritt einen Ohrabdruck (Positiv) zur Herstellung einer Otoplastik (für hinter dem Ohr getragene Geräte) oder einer Schale (für im Ohr getragene Geräte). In einem zweiten Schritt wird mittels der Abformung eine Negativform (N) angefertigt, in die nachfolgend entweder eine strahlungshärtbare oder eine autopolymerisierende, niedrig-viskose Formulierung gegossen wird. Danach wird z.B. mittels Hitze in einem Drucktopf oder mittels Strahlung ausgehärtet.

In der Druckschrift EP 0410 034 A1 ist ein Verfahren zur Herstellung einer Gehäuseschale eines im Ohr zu tragenden Hörgerätes beschrieben, welches zunächst die Herstellung eines positiven Ohrabdrucks sowie die anschließende Herstellung einer Negativform umfasst. In die Negativform wird ein Kunststoff gegossen, welcher durch Polymerisation aushärtet. Die so gefertigte Dentalschiene (Negativ) oder das Ohrpassstück (Positiv) müssen den anatomischen Gegebenheiten optimal angepasst sein. Andernfalls würden ungenaue Passstücke Beschwerden (z. B. Druckstellen, schlechter Halt) verursachen und die Funktion von Schiene oder Hörgerät beeinträchtigen (z. B. Zahnfehlstellungen/Rückkopplungen). Demzufolge ist es wichtig, dass die Formulierung möglichst niedrigviskos ist, so dass auch Unterschnitte und feinste Oberflächentexturen vom Material ausgefüllt und möglichst detailgetreu abgebildet werden können.

Ein Zweikomponentensystem zur Herstellung eines hypoallergenen schlagzähen Dentalbasiskunststoffes ist in DE 10 2012 022 693 A1 beschrieben. Das Zweikomponentensystem umfasst als feste Komponente A Polymerisate von mono- oder mehrfunktionellen Methacrylaten und als flüssige Komponente B polymerisierbare Methacrylate.

Ein weiches Unterfüllungsmaterial für eine Zahnprothesengrundlage wird in DE 10147 125 A1 offenbart. Die Harzzusammensetzung umfasst (Meth)acrylat-Monomere, Weichmacher, (Meth)acrylat-Polymere oder -Copolymere und einen Polymerisationsstarter.

Als weitere Verfahrensgruppe des Standes der Technik zur Herstellung von Schienen/Ohrpassstücken, die auf der Basis digitaler Daten funktioniert, kommen Schichtbauverfahren wie z. B. die Stereolithographie zum Einsatz. Dabei ist aus der Druckschrift US 4,575,330 bekannt, dass niedrigviskose, strahlungshärtbare Harze bzw. Harzgemische für die Herstellung von dreidimensionalen Objekten mittels Stereolithographie eingesetzt werden können. Ferner ist aus den Druckschriften US 5,487,012 und WO 01/87001 bekannt, dass die Stereolithographie vorteilhaft zur Herstellung von Ohrstücken eingesetzt werden kann.

Beim stereolithographischen Verfahren werden dreidimensionale Objekte aus einer niedrigviskosen, strahlungshärtbaren Formulierung in der Weise aufgebaut, dass jeweils eine dünne Schicht (ca. 25 - 100 µm) der Formulierung mittels aktinischer Strahlung in definierter Weise so vorgehärtet wird, dass die erzeugte Schicht die gewünschte Querschnittsform des Objektes an dieser Stelle vorweist. Zeitgleich wird die erzeugte Schicht an die im Schritt zuvor gehärtete Schicht polymerisiert. Der Aufbau des Gesamtobjektes lässt sich so mit Hilfe eines computergesteuerten Lasersystems wie z.B. eines Nd:YVo₄ Festkörperlasers (Viper si² SLA System, 3D Systems, USA) bewerkstelligen. Der generierte Formkörper wird gegebenenfalls, z.B. durch Strahlung, nachgehärtet.

An die im stereolithographischen Prozess einsetzbaren Harzformulierungen werden besondere Anforderungen gestellt. Dabei sind insbesondere die Strahlungsempfindlichkeit und die Viskosität der Harzformulierungen, sowie die Festigkeit der mittels Laserhärtung vorgehärteten Formkörper zu nennen. Dieser nicht völlig gehärtete Formkörper wird in der Technik der Stereolithographie als Grünling bezeichnet, und die Festigkeit dieses Grünlings, charakterisiert durch den E-Modul und die Biegefestigkeit, bezeichnet man als Grünfestigkeit. Die Grünfestigkeit stellt für die Praxis der Stereolithographie einen wichtigen Parameter dar, da Formkörper mit geringer Grünfestigkeit sich während des Stereolithographieprozesses unter ihrem eigenen Gewicht deformieren oder während der Nachhärtung, beispielsweise mit einer Xenonbogen- oder Halogenlampe, absacken oder sich durchbiegen können.

Ferner werden verfahrensbedingt die Grünlinge auf unterstützenden Strukturen, sogenannten Supports, gebaut. Diese Supports müssen den Grünling stabil während des gesamten Herstellprozesses positionieren, da sich die Position der Grünlinge nicht durch den Beschichtungsprozesses verändern darf. Entsprechend dürfen die Supports für einen stereolithographischen Prozess nur eine minimale Flexibilität aufweisen.

Aus all diesen Gründen ist es nur sehr eingeschränkt möglich, flexible Ohrpassstücke auf der Basis 3-dimensionaler Daten zu generieren. Zum einen ist es für das stereolithographische Verfahren notwendig, möglichst niedrigviskose Harze (< 3 Pas) einzusetzen. Aus diesem Grunde sind gewisse Materialklassen, wie z.B. Silikonmaterialien oder hochgefüllte Composite, nicht oder nur sehr eingeschränkt zugänglich.

Dies gilt ebenso für Systeme, die einen so genannten temperaturinduzierten Memoryeffekt aufweisen. Dieser Effekt ist jedoch für viele medizintechnische Anwendungen nützlich und gerade für neuartige Anwendungen essentiell. Beispielsweise kann eine Dentalschiene beim Einsetzen in den Mund verdrillt werden. Durch den durch Körperwärme induzierten Memoryeffekt wird diese dann in die optimale Position während des Tragens zurückgeformt. Dies erhöht entscheidend den Tragekomfort und vermeidet, dass im Vergleich zu einem harten, verformten Material Fehlstellungen erzeugt werden.

Der Erfindung liegt daher die Aufgabe zugrunde, ein Rohling sowie ein Verfahren zur Herstellung eines Rohlings bereitzustellen, mittels dem in einfacher Weise medizintechnische Formteile, insbesondere Dentalschienen oder Ohrpassstücke besonders präzise hergestellt werden können, die darüber hinaus einen temperaturinduzierten Memoryeffekt aufweisen.

Die Aufgabe wird erfindungsgemäß durch einen Rohling gemäß Anspruch 1 sowie ein Verfahren gemäß Anspruch 13 gelöst. Vorteilhafte Weiterbildungen der Erfindung sind in den abhängigen Ansprüchen angegeben.

Der erfindungsgemäße Fräsrohling aus einem aus mindestens zwei Komponenten hergestellten Werkstoff zur Herstellung von medizintechnischen Formteilen, insbesondere von Dentalschienen oder Ohrpassstücken, weist als eine erste Komponente A ein Polyethylmethacrylat-Polymer (PEMA) oder ein Polyethylmethacrylat-Polymethylmethacrylat-Copolymer (PEMA-PMMA), insbesondere Polyalkylmethacrylat-Polymerpulver, und als eine zweite Komponente B mindestens ein Alkylacrylat- und/oder einer Alkylmethacrylat-Monomer, in dem das Polyethylmethacrylat-Polymer (PEMA) oder ein Polyethylmethacrylat-Polymethylmethacrylat-Copolymer (PEMA-PMMA)der Komponente A wenigstens teilweise, bevorzugt vollständig lösbar ist, wobei das Alkylacrylat- und/oder Alkylmethacrylat-Monomer der Komponente B wenigstens ein Monomer der Gruppe Methylmethacrylat, Ethylmethacrylat, Ethylacrylat, Ethoxyethylacrylat, Tetrahydrofurfurylmethacrylat, Tetrahydrofurfurylacrylat, Isobornylacrylat, Isobornylmethacrylat und Ethoxyethylmethacrylat ist. Der erfindungsgemäße Fräsrohling ist dadurch gekennzeichnet, dass
der Werkstoff als weiteren Bestandteil einen Flexibilisierer umfasst, wobei der Flexibilisierer aus der Gruppe der zitronensäurebasierten, der adipinsäurebasierten, der phthalsäurebasierten oder der aliphatischen Ester, insbesondere bevorzugt 1,2-Cyclohexandicarbonsäurediisononylester, ist und wobei
der Flexibilisierer in einer Konzentration von wenigstens 5 Massenprozent vorliegt, und
die Komponente A als Katalysator ein organisches Peroxid oder Barbitursäure bzw. ein Barbitursäure -Derivat umfasst, und
der Werkstoff einen temperaturabhängigen Memoryeffekt aufweist, sodass das Material nach einer Deformation beim Aufheizen in seine ursprüngliche Form zurückkehrt, und das Massenverhältnis der Komponenten A und B 0,5 - 2 beträgt.

Unter dem Aspekt, dass heutzutage in zunehmendem Maße biometrische Daten für eine Vielzahl von medizintechnischen Anwendungen zur Verfügung stehen und für solche Materialien noch kein digitaler Workflow zugänglich ist, ermöglicht es die Erfindung in vorteilhafter Weise besonders einfach spanbare Fräsrohlingen für z.B. dentale bzw. kieferorthopädische Schienen oder Ohrpassstücke, insbesondere auf der Basis von 3-dimensionalen Daten zur Verfügung zu stellen, bei denen die Oberfläche der Rohlinge während des Fräsens nicht verschmiert und das Endprodukt einen von der Temperatur abhängigen Memoryeffekt besitzt. Somit wird gewährleistet, dass z.B. das Ohrpassstück bei Raumtemperatur im hartelastischen Zustand in den Gehörgang eingesetzt werden kann und anschließend die durch das Einsetzen (meistens Eindrehen) induzierte Deformation des Ohrpassstückes durch Körperwärme wieder in den Urzustand zurückkehrt.

Grundsätzlich wird unter einem Fräsrohling ein Körper aus einem fräsbaren Werkstoff verstanden, der zunächst eine beliebige Form aufweisen kann. Bevorzugt ist der Körper dabei frei von Hohlräumen und/oder stofflich homogen gebildet. Weiterhin bevorzugt weist der Körper eine glatte Oberfläche und/oder eine kompakte Form auf.

Unter einem aus mindestens zwei Komponenten hergestellten Werkstoff ist ein Material zu verstehen, das durch die Mischung der Komponenten gebildet wird, insbesondere durch eine chemische Reaktion von jeweils mindestens einer in den mindestens zwei Komponenten enthaltenen Substanz. Dabei kann jeder der wenigstens zwei Komponenten aus einer einzelnen Substanz bestehen oder durch ein Stoffgemisch gebildet sein.

Bevorzugt ist die Komponente A im Wesentlichen fest und/oder die Komponente B im Wesentlichen flüssig, so dass die Komponente A besonders leicht in der flüssigen Komponente B gelöst werden und dabei zu einem Gießwerkstoff verarbeitet werden kann.

Unter dem Lösen einer Komponente in der anderen, insbesondere der Komponente A in der Komponente B, wird grundsätzlich jeder Prozess verstanden, bei dem sich die Substanzen und/oder Partikel der einen Komponente gleichmäßig in der anderen Komponente verteilen. Insbesondere ist in diesem Zusammenhang auch das Erzeugen einer feinen Dispersion bzw. Suspension als Lösen zu verstehen.

Bei dem Polyalkylmethacrylat-Polymer kann es sich grundsätzlich um jedes Polymer handeln, dessen Monomere ein Alkylmethacrylat umfassen. Dies schließt grundsätzlich auch sämtliche Copolymere ein. Bevorzugt ist das Polyalkylmethacrylat-Polymer jedoch ausschließlich aus Alkylmethacrylat-Monomeren gebildet.

Unter einem Polymerpulver wird ein feines, körniges Gemenge des Polymers verstanden, wobei die mittlere Korngröße bevorzugt kleiner als 1 mm, besonders bevorzugt kleiner als 500 µm und ganz besonders bevorzugt kleiner als 100 µm ist.

Als Monomer wird grundsätzlich jede chemische Substanz verstanden, die miteinander oder unter Zugabe eines Hilfsstoffes zur Polymerisation gebracht werden kann. Grundsätzlich kann es sich bei einem Monomer auch bereits um ein Dimer oder ein Oligomer einer Substanz handeln, die weiterhin polymerisationsfähig sind.

Bei dem Flexibilisierer kann es sich zunächst um einen beliebigen, aus dem Stand der Technik bekannten Flexibilisierer bzw. Weichmacher handeln.

Nach einer vorteilhaften Ausgestaltung der Erfindung umfasst die Komponente A mindestens ein Polyethylmethacrylat-Polymer (PEMA) oder ein Polyethylmethacrylat-Polymethylmethacrylat-Copolymer (PEMA-PMMA), insbesondere mindestens ein PEMA- Pulver oder PEMA-Copolymer-Pulver, wodurch der Gefäßrohlings in besonders einfacher Weise und besonders kostengünstig hergestellt werden kann und darüber hinaus für medizintechnische Formteile günstige Eigenschaften aufweist. Weiterhin können auch andere Polymerpulver wie z.B. PMMA (Polymethylmethacrylat) in Kombination mit dem PEMA-Polymerpulver eingesetzt werden.

Gemäß einer bevorzugten Weiterbildung der Erfindung besteht die Komponente A zu mindestens 50 %, bevorzugt 60 % und besonders bevorzugt 70 % bezogen auf die Masse der Komponente A aus Polyethylmethacrylat-Polymer oder -Copolymer.

Gemäß einer besonders bevorzugten Weiterbildung der Erfindung ist die Komponente A bis auf einen geringen Anteil an Zusatzstoffen, insbesondere zu mindestens 90 % und besonders bevorzugt zu mindestens 95 % bezogen auf die Masse der Komponente A aus Polyethylmethacrylat-Polymer oder Polyethylmethacrylat-Copolymer gebildet.

Gemäß einer bevorzugten Ausgestaltung der Erfindung beträgt der Massenanteil des Polymerpulvers aus Polyethylmethacrylat-Polymer oder Polyethylmethacrylat-Polymethylmethacrylat-Copolymer an der Gesamtmasse des Fräsrohlings mindestens 25 %, bevorzugt mindestens 45 % und ganz besonders bevorzugt mindestens 50 %.

Nach einer vorteilhaften Weiterbildung der Erfindung umfasst die Komponente A als einen Katalysator ein organisches Peroxid wie Benzoylperoxid und/oder Toluidin, oder Barbitursäure bzw. ein Barbitursäure -Derivat, wobei der Katalysator bevorzugt in einer Konzentration von 0,25 - 1 Massenprozent vorliegt. Das Beimengen eines Katalysators verbessert dabei in vorteilhafter Weise die Polymerisationsfähigkeit der in der Komponente B enthaltenen Monomere.

In den erfindungsgemäßen Formulierungen ist nach einer Weiterbildung ein Katalysator bevorzugt, der zur Gruppe der Barbitursäuren und deren Derivaten gehört. Dabei sind die in der folgenden nicht einschränkenden Liste von Barbitursäure-Derivaten genannten Substanzen besonders gut anwendbar: Barbitursäure, 1,3-Dimethylbarbitursäure, 1,3-Diphenylbarbitursäure, 1,5-Dimethylbarbitursäure,5-Butylbarbitursäure, 5-Ethylbarbitursäure, 5-Isopropylbarbitursäure, 5-Cyclohexylbarbitursäure, 1,3,5-Trimethylbarbitursäure, 1,3-Dimethyl-5-ethylbarbitursäure, 1,3-Dimethyl-n-butylbarbitursäure, 1,3-Dimethyl-5-isobutylbarbitursäure, 1,3-Dimethyl-5-tert-butylbarbitursäure, 1,3-Dimethyl-5-cyclopentylbarbitursäure, 1,3-Dimethyl-5-cyclohexylbarbitursäure, 1,3-Dimethyl-5-phenylbarbitursäure, 1-Cyclohexyl-5-ethylbarbitursäure, 1-Benzyl-5-phenylbarbitursäure und Thiobarbitursäuren sowie deren Salze. Diese Verbindungen und Anwendungen werden in den Druckschriften US 5,707,611, US 5,663,214, US 4,906,446 und US 4,115,346 beschrieben.

Gemäß einer bevorzugten Ausgestaltung der Erfindung ist das Alkylacrylat- und/oder Alkylmethacrylat-Monomer der Komponente B wenigstens ein Monomer der Gruppe Methylmethacrylat, Ethylmethacrylat, Ethylacrylat, Ethoxyethylacrylat, Tetrahydrofurfurylmethacrylat, Tetrahydrofurfurylacrylat, Isobornylacrylat und/oder Isobornylmethacrylat und bevorzugt Ethoxyethylmethacrylat. Grundsätzlich ist die Liste der Alkylacrylat- und/oder Alkylmethacrylat-Monomer jedoch als nicht einschränkend zu betrachten. Diese können auch in Kombination verwendet und/oder mit weiteren Verbindungen aus der Gruppe der Acrylate oder Methacrylate formuliert werden. Dazu zählen beispielsweise: Bisphenol-A-ethoxylat(2)dimethacrylat, Bisphenol-A-ethoxylat(4)dimethacrylat, Bisphenol-A-propoxylat(2)dimethacrylat, Bisphenol-A-propoxylat(4)dimethacrylat sowie Dimethacrylate des (n)-alkoxylierten Bisphenol F wie Bisphenol-F-ethoxylat(2)dimethacrylat und Bisphenol-F-ethoxylat(4)dimethacrylat, Bisphenol-F-propoxylat(2)dimethacrylat, Bisphenol-F-propoxylat(4)dimethacrylat und Mischungen dieser. Vorzugsweise verwendet man monomere oder oligomere Dimethacrylate auf Basis von Bisphenol A, insbesondere das Bisphenol-A-ethoxylat(2)dimethacrylat und das Bisphenol-A-ethoxylat(4) dimethacrylat. Diese Liste ist ebenfalls als nicht einschränkend zu betrachten.

Gemäß einer vorteilhaften Weiterbildung der Erfindung umfasst die Komponente B wenigstens 5 %, bevorzugt wenigstens 10 %, besonders bevorzugt wenigstens 20 % bezogen auf die Masse der Komponente B des Alkylacrylat- und/oder Alkylmethacrylat-Monomers, wodurch in besonders einfacher Weise eine gute und vollständige Lösbarkeit des Polymer der Komponente A erreicht wird.

Nach einer bevorzugten Ausgestaltung der Erfindung umfasst die Komponente B wenigstens zwei verschiedene Alkylacrylat- und/oder Alkylmethacrylat-Monomere, die bevorzugt einen gemeinsamen Massenanteil von wenigstens 50 %, besonders bevorzugt 70 % und ganz besonders bevorzugt 80 % bezogen auf die Masse der Komponente B haben, wodurch die Eigenschaften des Fräsrohlings in besonders einfacher Weise eingestellt werden können und eine besonders kostengünstige Herstellung möglich ist. Gemäß einer weiteren Ausgestaltung der Erfindung beträgt der Massenanteil des Weichmachers bzw. Flexibilisierers wenigstens 5 Massenprozent, bevorzugt wenigstens 10 Massenprozent und besonders bevorzugt wenigstens 15 Massenprozent. Mittels des Flexibilisierers kann in besonders einfacher Weise der thermoelastische Bereich des ausgehärteten Endproduktes in Richtung Körpertemperatur verschoben werden. Der Massenanteil des Flexibilisierers kann sich grundsätzlich sowohl auf die Gesamtmasse des Werkstoffes als auch auf die Masse einer der beiden Komponenten A oder B beziehen. Besonders bevorzugt wird der Flexibilisierer jedoch der Komponente B zugesetzt bzw. ist in dieser Komponente enthalten, bevor diese mit der Komponente A vermischt wird. Wenn der Massenanteil dann lediglich auf die Komponente B bezogen wird, liegt der Massenanteil des Flexibilisierers bezogen auf die Gesamtmasse des Werkstoffs auch somit unter 5 Massenprozent, abhängig von dem Mischungsverhältnis der Komponenten A und B. Durch die Zugabe des Flexibilisierers als weiterer Bestandteil wird eine besonders einfache Herstellung des Fräsrohlings ermöglicht. Ganz besonders bevorzugt enthält der Werkstoff lediglich die Komponente A sowie die den Flexibilisierer enthaltende Komponente B.

Nach einer ebenfalls bevorzugten Ausgestaltung der Erfindung ist der Flexibilisierer aus der Gruppe der Phthalsäureester wie Diethylhexylphthalat (DEHP), der Gruppe der aliphatischen Ester, insbesondere der Gruppe der Alkylsulfonsäureester des Phenols, der Gruppe der zitronensäurebasierten Flexibilisierer wie Zitronensäuretriethylester oder besonders bevorzugt 1,2-Cyclohexandicarbonsäurediisononylester, oder der adipinsäurebasierten Flexibilisierer wie Diethylhexyladipat oder Diethyloctyladipat ausgewählt.

Im Sinne der Erfindung und mit Blick auf den medizintechnischen Anwendungsbereich werden Flexibilisierer aus der Gruppe der aliphatischen Ester wie 1,2-Cyclohexandicarbonsäurediisononylester bevorzugt (siehe dazu auch A. Gärtner: "Weichmacher (DEHP) in Medizinprodukten"; mt-medizintechnik; 3/2007; TUEV Media Verlag Köln, S. 92-102).

Gemäß einer bevorzugten Ausgestaltung der Erfindung beträgt das Massenverhältnis der Komponente A zur Komponente B 0,5 - 2, bevorzugt 0,65 - 1,5 und besonders bevorzugt 0,75 - 1, wodurch zugleich die erwünschten Materialeigenschaften und eine besonders günstige Herstellung erreicht werden.

Nach einer vorteilhaften Weiterbildung der Erfindung weist der Werkstoff einen temperaturabhängigen Memoryeffekt auf, so dass das Material, insbesondere einer fertigen Dentalschiene oder eines Ohrpassstückes, nach einer Deformation durch Aufheizen, insbesondere auf eine Temperatur von 37 °C in seine ursprüngliche Form zurückkehrt.

Weiterhin betrifft die Erfindung ein Verfahren zur Herstellung eines Fräsrohlings aus einem Werkstoff für medizintechnische Formteile, wobei zunächst jeweils
- eine Komponente A umfassend ein Polyethylmethacrylat-Polymer (PEMA) oder ein Polyethylmethacrylat-Polymethylmethacrylat-Copolymer (PEMA-PMMA) und einen Katalysator, wobei der Katalysator ein organisches Peroxid oder Barbitursäure bzw. ein Barbitursäure-Derivat ist, und
- eine Komponente B umfassend wenigstens ein Alkylacrylat- und/oder ein Alkylmethacrylat- Monomer, in dem das Polyethylmethacrylat-Polymer (PEMA) oder ein Polyethylmethacrylat-Polymethylmethacrylat-Copolymer (PEMA-PMMA) der Komponente A wenigstens teilweise, bevorzugt vollständig lösbar ist, wobei das Alkylacrylat- und/oder Alkylmethacrylat-Monomer der Komponente B wenigstens ein Monomer der Gruppe Methylmethacrylat, Ethylmethacrylat, Ethylacrylat, Ethoxyethylacrylat, Tetrahydrofurfurylmethacrylat, Tetrahydrofurfurylacrylat, Isobornylacrylat, Isobornylmethacrylat und Ethoxyethylmethacrylat ist,
hergestellt wird, gefolgt von dem Mischen der Komponenten A, B und eines Flexibilisierers aus der Gruppe der zitronensäurebasierten, der adipinsäurebasierten, der phthalsäurebasierten oder der aliphatischen Ester, insbesondere bevorzugt 1,2-Cyclohexandicarbonsäurediisononylester, wobeider Flexibilisierer in einer Konzentration von wenigstens 5 Massenprozent vorliegt, und wobei das Massenverhältnis der Komponenten A und B 0,5 - 2 beträgt,
sowie der nachfolgenden Aushärtung der Mischung.

Das erfindungsgemäße Verfahren ermöglicht es in besonders einfacher Weise einen Fräsrohling herzustellen, wobei weder besondere Fachkenntnisse im Bereich der Polymerwissenschaften noch eine aufwändige, apparative Ausstattung notwendig sind.

Unter dem Herstellen einer Komponente wird grundsätzlich jede Form des Bereitstellens dieser Komponente verstanden. Dabei kann es sich um das Mischen mehrerer Einzelbestandteile, das Aufbereiten einer oder mehrerer Bestandteile einer Komponente, beispielsweise durch Zerkleinern, oder aber das bloße Wiegen, Abmessen bzw. Entnehmen aus einer bereits vorbereiteten Einzelverpackung der benötigten Menge der jeweiligen Komponente bzw. Bestandteile einer Komponente handeln.

Nach einer vorteilhaften Weiterbildung des Verfahrens erfolgt das Aushärten bei einer Temperatur zwischen 30 °C und 70 °C, bevorzugt zwischen 40 °C und 60 °C und besonders bevorzugt zwischen 45 °C und 55 °C und/oder über eine Dauer von 20 - 90 Minuten, bevorzugt 30 - 60 Minuten und besonders bevorzugt 45 Minuten, wodurch ohne großen Energieaufwand ein gebrauchsfertiger Fräsrohling erhalten werden kann.

Nach einer weiteren vorteilhaften Ausgestaltung des Verfahrens erfolgt das Aushärten in einem Drucktopf bei einem Innendruck von wenigstens 3 bar, bevorzugt wenigstens 5 bar, wodurch eine zügige und blasenfreie Aushärtung gewährleistet wird.

Ein Ausführungsbeispiel der Erfindung wird nachstehend näher erläutert. In der Figur zeigt:

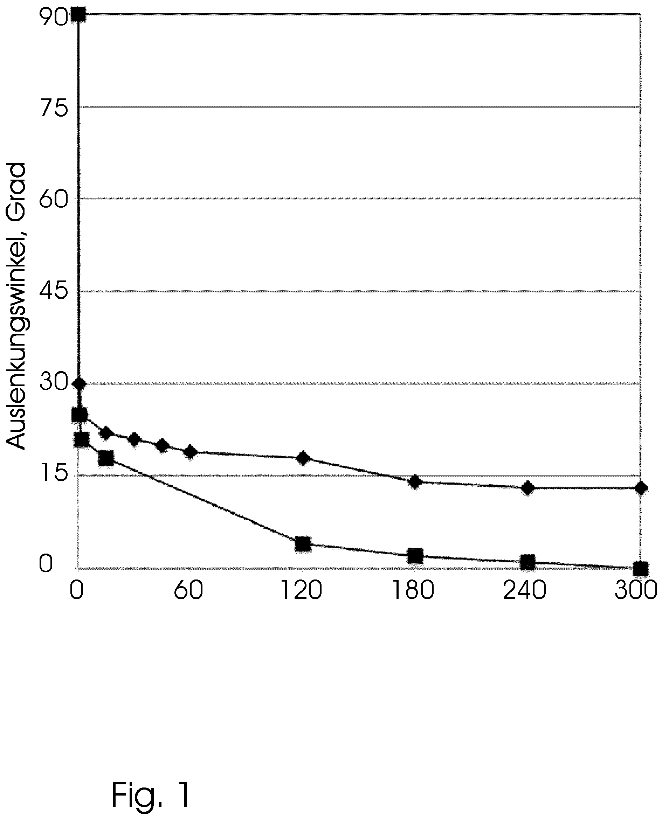

- Fig. 1: Zeitabhängige Relaxation von Prüfkörpern (5 x 5 x 80 mm) bei 23°C (◆) und bei 37°C (■)

Zur Herstellung eines Fräsrohlings zur Herstellung von medizintechnischen Formteilen wird ein aus zwei Komponenten A und B hergestellter Werkstoff verwendet, deren Zusammensetzungen in der nachfolgenden Tabelle dargestellt sind.

| Komponente A | Massenanteil, % |
|---|---|
| Polyethylmethacrylat | 99 |
| 1-Benzyl-5-phenylbarbitursäure | 1 |
| | |

| Komponente B | Massenanteil, % |
|---|---|
| Ethoxyethylmethacrylat | 49,73 |
| Tetrahydrofurfurylmethacrylat | 33,2 |
| 1,2 Cyclohexandicarboxylsäurediisononylester | 16,5 |
| Dilauryldimethylammoniumchlorid | 0,4 |
| 1% Kupfer(II)-acetylacetonat Lösung in MMA | 0,17 |

Die beiden erfindungsgemäßen Komponenten werden im Verhältnis A : B = 100 : 75 mit einem Spatel in einem Becherglas gemischt und anschließend in einem Drucktopf (Polymax, Fa. Dreve) bei 50°C und 6 bar für 45 min blasenfrei in einer Dubliersilikonform ausgehärtet. Dabei wird ein Fräsrohling mit einem Durchmesser von 98 mm und einer Dicke von 18 mm erhalten.

Aus diesem Fräsrohling werden dann Prüfkörper der Dimension 5x5x80 mm mittels einer Fräsmaschine (Otofab 1, Fa. pro3dure) generiert. Alternativ erfolgt die Herstellung von medizintechnischen Formteilen auf der Basis von 3-dimensionalen Daten.

Die Prüfkörper werden dann bei 23 °C und bei 37 °C für 24h temperiert und anschließend mittig um 90° gebogen. Die Relaxation des Winkels wird nachfolgend zeitabhängig bei den beiden oben genannten Temperaturen aufgezeichnet, um den temperaturabhängigen Memoryeffekt zu dokumentieren. Die Ergebnisse sind in Figur 1 dargestellt. Dabei zeigt sich, dass die erfindungsgemäße Formulierung einen temperaturabhängigen Memoryeffekt aufweist mittels dessen Formkörper, die bei Raumtemperatur verformt werden, durch Körperwärme nahezu in ihre Ursprungsform zurückkehren.

## Patentansprüche

1. Fräsrohling aus einem aus mindestens zwei Komponenten hergestellten Werkstoff zur Herstellung von medizintechnischen Formteilen, wobei eine erste Komponente A ein Polyethylmethacrylat-Polymer (PEMA) oder ein Polyethylmethacrylat-Polymethylmethacrylat-Copolymer (PEMA-PMMA), und eine zweite Komponente B mindestens ein Alkylacrylat- und/oder ein Alkylmethacrylat-Monomer, in dem das Polyethylmethacrylat-Polymer (PEMA) oder das Polyethylmethacrylat-Polymethylmethacrylat-Copolymer (PEMA-PMMA) der Komponente A wenigstens teilweise lösbar ist, umfasst, wobei das Alkylacrylat- und/oder Alkylmethacrylat-Monomer der Komponente B wenigstens ein Monomer der Gruppe Methylmethacrylat, Ethylmethacrylat, Ethylacrylat, Ethoxyethylacrylat, Tetrahydrofurfurylmethacrylat, Tetrahydrofurfurylacrylat, Isobornylacrylat, Isobornylmethacrylat und Ethoxyethylmethacrylat ist,
**dadurch gekennzeichnet, dass**
der Werkstoff als weiteren Bestandteil einen Flexibilisierer umfasst, wobei der Flexibilisierer aus der Gruppe der zitronensäurebasierten, der adipinsäurebasierten, der phthalsäurebasierten oder der aliphatischen Ester, insbesondere bevorzugt 1,2-Cyclohexandicarbonsäurediisononylester, ist und wobei
der Flexibilisierer in einer Konzentration von wenigstens 5 Massenprozent vorliegt, und
die Komponente A als Katalysator ein organisches Peroxid oder Barbitursäure bzw. ein Barbitursäure -Derivat umfasst, und
der Werkstoff einen temperaturabhängigen Memoryeffekt aufweist, sodass das Material nach einer Deformation beim Aufheizen in seine ursprüngliche Form zurückkehrt, und
das Massenverhältnis der Komponenten A und B 0,5 - 2 beträgt.

2. Fräsrohling nach Anspruch 1, **dadurch gekennzeichnet, dass** die Komponente A ein Polyethylmethacrylat-Polymer (PEMA)-Pulver oder ein Polyethylmethacrylat-Polymethylmethacrylat-Copolymer-Pulver (PEMA-PMMA) umfasst.

3. Fräsrohling nach wenigstens einem der vorhergehenden Ansprüche, **dadurch gekennzeichnet, dass** die Komponente A zu mindestens 50 %, bevorzugt 60 % und besonders bevorzugt 70 % bezogen auf die Masse der Komponente A aus Polyethylmethacrylat-Polymer oder -Copolymer besteht.

4. Fräsrohling nach wenigstens einem der vorhergehenden Ansprüche, **gekennzeichnet durch** einen Massenanteil von mindestens 25 % des Polymerpulvers aus Polyethylmethacrylat-Polymer oder Polyethylmethacrylat-Polymethylmethacrylat-Copolymer.

5. Fräsrohling nach wenigstens einem der vorhergehenden Ansprüche, wobei das organische Peroxid Benzoylperoxid und/oder Toluidin ist.

6. Fräsrohling nach wenigstens einem der vorhergehenden Ansprüche, **dadurch gekennzeichnet, dass** der Katalysator in einer Konzentration von 0,25 - 1 Massenprozent vorliegt.

7. Fräsrohling nach wenigstens einem der vorhergehenden Ansprüche, **dadurch gekennzeichnet, dass** das Alkylacrylat- und/oder Alkylmethacrylat-Monomer der Komponente B Ethoxyethylmethacrylat ist.

8. Fräsrohling nach wenigstens einem der vorhergehenden Ansprüche, **dadurch gekennzeichnet, dass** die Komponente B wenigstens 5 %, bevorzugt wenigstens 10 %, besonders bevorzugt wenigstens 20 %, bezogen auf die Masse der Komponente B, des Alkylacrylat- und/oder Alkylmethacrylat-Monomers umfasst.

9. Fräsrohling nach wenigstens einem der vorhergehenden Ansprüche, **dadurch gekennzeichnet, dass** die Komponente B wenigstens zwei verschiedene Alkylacrylat- und/oder Alkylmethacrylat-Monomere umfasst, die bevorzugt einen gemeinsamen Massenanteil von wenigstens 50 %, besonders bevorzugt 70 % und ganz besonders bevorzugt 80 % bezogen auf die Masse der Komponente B haben.

10. Fräsrohling nach wenigstens einem der vorhergehenden Ansprüche, **dadurch gekennzeichnet, dass** der Flexibilisierer in einer Konzentration von wenigstens 10 Massenprozent, bevorzugt wenigstens 15 Massenprozent vorliegt.

11. Fräsrohling nach wenigstens einem der vorhergehenden Ansprüche, **dadurch gekennzeichnet, dass** das Massenverhältnis der Komponenten A und B 0,65 - 1,5 und bevorzugt 0,75 - 1 beträgt.

12. Fräsrohling nach wenigstens einem der vorhergehenden Ansprüche, **gekennzeichnet durch** einen temperaturabhängigen Memoryeffekt des Werkstoffes, so dass das Material nach einer Deformation beim Aufheizen auf eine Temperatur von 37 °C in seine ursprüngliche Form zurückkehrt.

13. Verfahren zur Herstellung eines Fräsrohlings aus einem Werkstoff für medizintechnische Formteile, wobei der Werkstoff einen temperaturabhängigen Memoryeffekt aufweist, so dass das Material nach einer Deformation beim Aufheizen in seine ursprüngliche Form zurückkehrt, umfassend die Schritte:
- Herstellen einer Komponente A umfassend ein Polyethylmethacrylat-Polymer (PEMA) oder ein Polyethylmethacrylat-Polymethylmethacrylat-Copolymer (PEMA-PMMA) und einen Katalysator, wobei der Katalysator ein organisches Peroxid oder Barbitursäure bzw. ein Barbitursäure-Derivat ist,
- Herstellen einer Komponente B umfassend wenigstens ein Alkylacrylat- und/oder ein Alkylmethacrylat-Monomer, in dem das Polyethylmethacrylat-Polymer (PEMA) oder ein Polyethylmethacrylat-Polymethylmethacrylat-Copolymer (PEMA-PMMA) der Komponente A wenigstens teilweise lösbar ist, wobei das Alkylacrylat- und/oder Alkylmethacrylat-Monomer der Komponente B wenigstens ein Monomer der Gruppe Methylmethacrylat, Ethylmethacrylat, Ethylacrylat, Ethoxyethylacrylat, Tetrahydrofurfurylmethacrylat, Tetrahydrofurfurylacrylat, Isobornylacrylat, Isobornylmethacrylat und Ethoxyethylmethacrylat ist,
- Mischen der Komponenten A und B sowie eines Flexibilisierers, wobei der Flexibilisierer aus der Gruppe der zitronensäurebasierten, der adipinsäurebasierten, der phthalsäurebasierten oder der aliphatischen Ester, insbesondere bevorzugt 1,2-Cyclohexandicarbonsäurediisononylester, ist,
wobeider Flexibilisierer in einer Konzentration von wenigstens 5 Massenprozent vorliegt, und wobei
das Massenverhältnis der Komponenten A und B 0,5 - 2 beträgt,
- Aushärten der Mischung.

14. Verfahren zur Herstellung eines Fräsrohlings nach Anspruch 13, **dadurch gekennzeichnet, dass** das Aushärten bei einer Temperatur zwischen 30 °C und 70 °C, bevorzugt zwischen 40 °C und 60 °C und besonders bevorzugt zwischen 45 °C und 55 °C und/oder über eine Dauer von 20 - 90 Minuten, bevorzugt 30 - 60 Minuten und besonders bevorzugt 45 Minuten erfolgt.

15. Verfahren zur Herstellung eines Fräsrohlings nach Anspruch 13 oder 14, **dadurch gekennzeichnet, dass** das Aushärten in einem Drucktopf bei einem Innendruck von wenigstens 3 bar, bevorzugt wenigstens 5 bar erfolgt.

## Claims

1. Milling blank made of a material produced from at least two components for producing medical-technical molded parts, wherein
a first component A comprises a polyethyl methacrylate polymer (PEMA) or a polyethyl methacrylate-polymethyl methacrylate copolymer (PEMA-PMMA), and
a second component B comprises at least one alkyl acrylate and/or one alkyl methacrylate monomer in which the polyethyl methacrylate polymer (PEMA) or the polyethyl methacrylate-polymethyl methacrylate copolymer (PEMA-PMMA) of component A is at least partially soluble, wherein the alkyl acrylate and/or alkyl methacrylate monomer of component B is at least one monomer of the group methyl methacrylate, ethyl methacrylate, ethyl acrylate, ethoxyethyl acrylate, tetrahydrofurfuryl methacrylate, tetrahydrofurfuryl acrylate, isobornyl acrylate, isobornyl methacrylate and ethoxyethyl methacrylate,
**characterized in in that**
the material comprises a flexibiliser as a further component, wherein the flexibiliser is from the group of citric acid-based, adipic acid-based, phthalic acid-based or aliphatic esters, in particular preferably 1,2-cyclohexanedicarboxylic acid diisononyl esters, and wherein
the flexibilizer is present in a concentration of at least 5 mass percent, and component A comprises as catalyst an organic peroxide or barbituric acid or a barbituric acid derivative, and
the material has a temperature-dependent memory effect so that the material returns to its original shape after deformation during heating, and
the mass ratio of components A and B is 0.5 - 2.

2. Milling blank according to claim 1, **characterized in that** component A comprises a polyethyl methacrylate polymer (PEMA) powder or a polyethyl methacrylate-polymethyl methacrylate copolymer powder (PEMA-PMMA).

3. Milling blank according to at least one of the preceding claims, **characterized in that** component A consists of at least 50%, preferably 60% and particularly preferably 70% of polyethyl methacrylate polymer or copolymer based on the mass of component A.

4. Milling blank according to at least one of the preceding claims, **characterized by** a mass fraction of at least 25% of the polymer powder made of polyethyl methacrylate polymer or polyethyl methacrylate-polymethyl methacrylate copolymer.

5. Milling blank according to at least one of the preceding claims, wherein the organic peroxide is benzoyl peroxide and/or toluidine.

6. Milling blank according to at least one of the preceding claims, **characterized in that** the catalyst is present in a concentration of 0.25 - 1 mass percent.

7. Milling blank according to at least one of the preceding claims, **characterized in that** the alkyl acrylate and/or alkyl methacrylate monomer of component B is ethoxyethyl methacrylate.

8. Milling blank according to at least one of the preceding claims, **characterized in that** component B comprises at least 5%, preferably at least 10%, particularly preferably at least 20% of alkyl acrylate and/or alkyl methacrylate monomer based on the mass of component B.

9. Milling blank according to at least one of the preceding claims, **characterized in that** component B comprises at least two different alkyl acrylate and/or alkyl methacrylate monomers, which preferably have a common mass fraction of at least 50%, particularly preferably 70% and very particularly preferably 80%, based on the mass of component B.

10. Milling blank according to at least one of the preceding claims, **characterized in that** the flexibilizer is present in a concentration of at least 10 mass percent, preferably at least 15 mass percent.

11. Milling blank according to at least one of the preceding claims, **characterized in that** the mass ratio of components A and B is 0.65 - 1.5 and preferably 0.75 - 1.

12. Milling blank according to at least one of the preceding claims, **characterized by** a temperature-dependent memory effect of the material, so that the material returns to its original shape after a deformation during heating to a temperature of 37 °C.

13. Method for producing a milling blank made of a material for medical-technical molded parts, wherein the material has a temperature-dependent memory effect, so that the material returns to its original shape after a deformation during heating, comprising the steps
- preparing a component A comprising a polyethyl methacrylate polymer (PEMA) or a polyethyl methacrylate-polymethyl methacrylate copolymer (PEMA-PMMA) and a catalyst, wherein the catalyst is an organic peroxide or barbituric acid or a barbituric acid derivative,
- preparing a component B comprising at least one alkyl acrylate and/or one alkyl methacrylate monomer in which the polyethyl methacrylate polymer (PEMA) or a polyethyl methacrylate-polymethyl methacrylate copolymer (PEMA-PMMA) of component A is at least partially soluble, wherein the alkyl acrylate and/or alkyl methacrylate monomer of component B is at least one monomer of the group methyl methacrylate, ethyl methacrylate, ethyl acrylate, ethoxyethyl acrylate, tetrahydrofurfuryl methacrylate, tetrahydrofurfuryl acrylate, isobornyl acrylate, isobornyl methacrylate and ethoxyethyl methacrylate,
- mixing components A and B and a flexibiliser, wherein the flexibiliser is from the group of citric acid-based, adipic acid-based, phthalic acid-based or aliphatic esters, in particular preferably 1,2-cyclohexanedicarboxylic acid diononyl esters,
wherein the flexibilizer is present in a concentration of at least 5 mass percent, and wherein
the mass ratio of components A and B is 0.5 - 2,
- curing of the mixture.

14. Method for producing a milling blank according to claim 13, **characterized in that** the curing is carried out at a temperature between 30°C and 70°C, preferably between 40°C and 60°C and particularly preferably between 45°C and 55°C and/or over a period of 20 - 90 minutes, preferably 30 - 60 minutes and particularly preferably 45 minutes.

15. Method for producing a milling blank according to claim 13 or 14, **characterized in that** the curing is carried out in a pressure pot at an internal pressure of at least 3 bar, preferably at least 5 bar.

## Revendications

1. Flan de fraisage à base d'un matériau à deux composants au moins destiné à la fabrication de pièces moulées à usage médical, dans lequel
un premier composant A renferme un polymère polyméthacrylate d'éthyle (PEMA) ou un copolymère de polyméthacrylate d'éthyle-polyméthacrylate de méthyle (PEMA-PMMA), et un second composant B renferme au moins un monomère d'acrylate d'alkyle et / ou de méthacrylate d'alkyle, dans lequel le polymère polyméthacrylate d'éthyle (PEMA) ou le copolymère de polyméthacrylate d'éthyle-polyméthacrylate de méthyle (PEMA-PMMA) du composant A est au moins en partie soluble,
dans lequel le monomère d'acrylate d'alkyle et / ou de méthacrylate d'alkyle du composant B est au moins un monomère du groupe méthacrylate de méthyle, méthacrylate d'éthyle, acrylate d'éthyle, acrylate d'éthyle éthoxylé, méthacrylate tétrahydrofurfurylique, acrylate tétrahydrofurfurylique, acrylate d'isobornyle, méthacrylate d'isobornyle et méthacrylate d'éthyle éthoxylé,
**caractérisé en ce que**
le matériau renferme un plastifiant (*agent assouplissant*) comme autre constituant, dans lequel le plastifiant est issu du groupe des esters à base d'acide citrique, à base d'acide adipique, à base d'acide phtalique ou aliphatique, notamment de l'ester diisononylique de l'acide cyclohexane-1,2-dicarboxylique et dans lequel le plastifiant est présent dans une concentration d'au moins 5 pour cent en masse, et le composant A renferme un peroxyde organique ou un acide barbiturique ou un dérivé d'acide barbiturique comme catalyseur, et
le matériau présente un effet de mémoire dépendant de la température de sorte que le matériau revient dans sa forme initiale en s'échauffant après une déformation, et
le rapport de masse des composants A et B s'élève à 0,5 - 2.

2. Flan de fraisage selon la revendication 1, **caractérisé en ce que** le composant A renferme une poudre de polymère polyméthacrylate d'éthyle (PEMA) ou une poudre de copolymère de polyméthacrylate d'éthyle-polyméthacrylate de méthyle (PEMA-PMMA).

3. Flan de fraisage selon l'une au moins des revendications précédentes, **caractérisé en ce que** le composant A est constitué de polymère ou de copolymère de polyméthacrylate d'éthyle à raison d'au moins 50 %, de préférence 60 % et encore mieux 70 % rapporté à la masse du composant A.

4. Flan de fraisage selon l'une au moins des revendications précédentes, **caractérisé par** une fraction massique d'au moins 25 % de poudre de polymère à base de polymère de polyméthacrylate d'éthyle ou de copolymère de polyméthacrylate d'éthyle-polyméthacrylate de méthyle.

5. Flan de fraisage selon l'une au moins des revendications précédentes, dans lequel le peroxyde organique est du peroxyde de benzoyle et / ou de la toluidine.

6. Flan de fraisage selon l'une au moins des revendications précédentes, **caractérisé en ce que** le catalyseur est présent dans une concentration de 0,25 - 1 pour cent en masse.

7. Flan de fraisage selon l'une au moins des revendications précédentes, **caractérisé en ce que** le monomère d'acrylate d'alkyle et / ou de méthacrylate d'alkyle du composant B est du méthacrylate d'éthyle éthoxylé.

8. Flan de fraisage selon l'une au moins des revendications précédentes, **caractérisé en ce que** le composant B renferme du monomère d'acrylate d'alkyle et / ou de méthacrylate d'alkyle à raison d'au moins 5 %, de préférence d'au moins 10 % et encore mieux d'au moins 20 % rapporté à la masse du composant B.

9. Flan de fraisage selon l'une au moins des revendications précédentes, **caractérisé en ce que** le composant B renferme au moins deux monomères différents d'acrylate d'alkyle et / ou de méthacrylate d'alkyle, qui ont de préférence une fraction massique commune d'au moins 50 %, encore mieux de 70 % et tout particulièrement de 80 % rapportée à la masse du composant B.

10. Flan de fraisage selon l'une au moins des revendications précédentes, **caractérisé en ce que** le plastifiant est présent dans une concentration d'au moins 10 pour cent en masse, de préférence d'au moins 15 pour cent en masse.

11. Flan de fraisage selon l'une au moins des revendications précédentes, **caractérisé en ce que** le rapport de masse des composants A et B s'élève à 0,65 - 1,5 et de préférence à 0,75 - 1.

12. Flan de fraisage selon l'une au moins des revendications précédentes, **caractérisé par** un effet de mémoire du matériau dépendant de la température, de sorte que le matériau revient dans sa forme initiale en s'échauffant à une température de 37 °C après une déformation.

13. Procédé de fabrication d'un flan de fraisage à base d'un matériau destiné des pièces moulées à usage médical, dans lequel le matériau présente un effet de mémoire dépendant de la température, de sorte que le matériau revient dans sa forme initiale en s'échauffant après une déformation, comprenant les étapes suivantes :
- la fabrication d'un composant A renfermant un polymère polyméthacrylate d'éthyle (PEMA) ou un copolymère de polyméthacrylate d'éthyle-polyméthacrylate de méthyle (PEMA-PMMA) et un catalyseur, dans lequel le catalyseur est un peroxyde organique ou un acide barbiturique ou un dérivé d'acide barbiturique,
- la fabrication d'un composant B renfermant au moins un monomère d'acrylate d'alkyle et / ou de méthacrylate d'alkyle, dans lequel le polymère polyméthacrylate d'éthyle (PEMA) ou un copolymère de polyméthacrylate d'éthyle-polyméthacrylate de méthyle (PEMA-PMMA) du composant A est au moins en partie soluble, dans lequel le monomère d'acrylate d'alkyle et / ou de méthacrylate d'alkyle du composant B est au moins un monomère du groupe méthacrylate de méthyle, méthacrylate d'éthyle, acrylate d'éthyle, acrylate d'éthyle éthoxylé, méthacrylate tétrahydrofurfurylique, acrylate tétrahydrofurfurylique, acrylate d'isobornyle, méthacrylate d'isobornyle et méthacrylate d'éthyle éthoxylé,
- le mélange des composants A et B ainsi que d'un plastifiant, dans lequel le plastifiant est issu du groupe des esters à base d'acide citrique, à base d'acide adipique, à base d'acide phtalique ou aliphatique, notamment de l'ester diisononylique de l'acide cyclohexane-1,2-dicarboxylique,
dans lequel le plastifiant est présent dans une concentration d'au moins 5 pour cent en masse, et dans lequel
le rapport de masse des composants A et B s'élève à 0,5 - 2,
- le durcissement du mélange.

14. Procédé de fabrication d'un flan de fraisage selon la revendication 13, **caractérisé en ce que** le durcissement s'effectue à une température comprise entre 30 °C et 70 °C, de préférence entre 40 °C et 60 °C et encore mieux entre 45 °C et 55 °C et / ou sur une durée de 20 - 90 minutes, de préférence de 30 - 60 minutes et encore mieux de 45 minutes.

15. Procédé de fabrication d'un flan de fraisage selon la revendication 13 ou 14, **caractérisé en ce que** le durcissement s'effectue dans un récipient sous pression soumis à une pression intérieure d'au moins 3 bars, de préférence d'au moins 5 bars.
